# EUROPEAN PATENT SPECIFICATION

(11) **EP 3 125 139 B1**
(45) Date of publication and mention of the grant of the patent: **26.09.2018**
(21) Application number: 16166981.7
(22) Date of filing: 26.04.2016
(51) Int. Cl.: G16H 20/30

(54) **METHOD AND DEVICE FOR EXERCISE RECORDING**
VERFAHREN UND VORRICHTUNG ZUM AUFZEICHNEN VON ÜBUNGEN
PROCÉDÉ ET DISPOSITIF D'ENREGISTREMENT D'EXERCICE

(30) Priority: 31.07.2015 CN 201510462845
(43) Date of publication of application: 01.02.2017
(73) Proprietor: Xiaomi Inc., Beijing 100085 (CN)
(72) Inventor: LIU, Huayijun, 100085 BEIJING (CN); WU, Ke, 100085 BEIJING (CN); CHEN, Tao, 100085 BEIJING (CN)
(74) Representative: Delumeau, François Guy

(56) References cited:
- WO-A1-2015/003211
- US-A1- 2010 035 727
- US-A1- 2011 054 358
- US-A1- 2013 116 852

## Description

### FIELD

The present disclosure generally relates to the field of computer technology, and more particularly, to a method and device for exercise recording.

### BACKGROUND

As people have enthusiasm for exercises, statistical service of exercise quantity provided by electrical device, such as a smart wear equipment, terminal or the like, has attracted more and more attention. When a user is doing physical exercises, he may carry an electrical device to record his action. The electrical device may count the steps of the user, walking distance, and the calorie he burns, etc. Document US 2013/116852 is considered the closest prior art and discloses a device for providing haptic effects on a haptic surface, the device comprising actuators enabling the determination of an object positioned on the haptic surface.

### SUMMARY

In view of the fact in the related art, a method and device are provided for exercise recording.

According to a first aspect, the invention relates to a method according to Claim 1 for exercise recording, which applies to a mattress that has pressure sensors distributed thereon.

In one embodiment, the step of determining the critical force points for the user on the mattress comprising: determining n critical force points for the user on the mattress according to the pressure of the pressure sensors when the user lies down the mattress, wherein n≥2; and determining all the critical force points for the user on the mattress according to the n critical force points.

In one embodiment, the step of determining n critical force points for the user on the mattress according to the pressure of the pressure sensors when the user lies down the mattress comprising:
if n=2, finding two critical force points which are of the longest distance from each other and where the pressure values of the sensors is not zero in the length of the mattress; and determining the force point thereof with the bigger pressure value of the pressure sensors to be the force point of head, and determining the force point thereof with the smaller pressure value of the pressure sensors to be the force point of feet;
if n=3, finding two critical force points which are of the longest distance from each other and where the pressure values of the sensors are not zero in the length of the mattress; determining the force point thereof with the bigger pressure value of the pressure sensors to be the force point of head, and determining the force point thereof with the smaller pressure value of the pressure sensors to be the force point of feet; and determining the force point which is of the largest pressure value of the pressure sensor to be the force point of hip;
if n=5, finding two critical force points which are of the longest distance from each other and where the pressure values of the sensors are not zero in the length of the mattress; determining the force point thereof with the bigger pressure value of the pressure sensors to be the force point of head, and determining the force point thereof with the smaller pressure value of the pressure sensors to be the force point of feet; determining the force point which is of the largest pressure value of the pressure sensor to be the force point of hip; finding two critical force points which are of the longest distance from each other where the pressure values of the sensors are not zero in the width of the mattress, and determining the two critical force points to be the force points of hands.

In one embodiment, if n=2 and the n critical force points are the force point of head and the force point of feet, the step of determining all the critical force points for the user on the mattress according to the n critical force points comprising: determining the ratio of leg to body for the user according to his information prestored in the terminal; determining the force point of hip according to the ratio of leg to body and the positions of the force point of head and the force point of feet; determining a predefined area of both sides of the distance from the force point of head to the force point of hip to be the force range of the arms; and determining the force point of head, the force points of arms, the force point of hip and the force point of feet to be all the critical force points for the user on the mattress.

In one embodiment, if n=3 and the n critical force points are the force point of head, the force point of feet and the force point of hip, the step of determining all the critical force points for the user on the mattress according to the n critical force points comprising: determining a predefined area of both sides of the distance from the force point of head to the force point of hip to be the force range of the arms; and determining the force point of head, the force points of arms, the force point of hip and the force point of feet to be all the critical force points for the user on the mattress.

In one embodiment, if n=5 and the n critical force points are the force point of head, the force point of feet, the force point of hip and the force points of hands, the step of determining all the critical force points for the user on the mattress according to the n critical force points comprising: determining the two ranges from the force points of hands to the force point of head to be the force points of arms; and determining the force point of head, the force points of arms, the force point of hip and the force point of feet to be all the critical force points for the user on the mattress.

In one embodiment, the step of sending the exercise information including the exercise status to the terminal including: generating exercise information including the exercise status, and sending the exercise information to the terminal, which determines the training parts of body of the user according to the exercise status; or acquiring the number of the movement, generating the exercise information including the exercise status and the number of movement , and sending the exercise information to the terminal , which computes total energy the user costs on the mattress according to the number of movement and the energy consumption of a single movement.

According to a second aspect, the invention relates to a device according to Claim 8 for exercise status recording, which applies to a mattress that has pressure sensors distributed thereon.

In one embodiment, the force point determining module comprising: a first determining module, used to determine n critical force points for the user on the mattress according to the pressure of the pressure sensors when the user lies down the mattress, wherein n≥2; and a second determining module, used to determine all the critical force points for the user on the mattress according to the n critical force points.

In one embodiment, the first determining module is further configured to:
if n=2, finding two critical force points which are of the longest distance from each other and where the pressure values of the sensors are not zero in the length of the mattress; and determining the force point thereof with the bigger pressure value of the pressure sensors to be the force point of head, and determining the force point thereof with the smaller pressure value of the pressure sensors to be the force point of feet;
if n=3, finding two critical force points which are of the longest distance from each other and where the pressure values of the sensors are not zero in the length of the mattress; determining the force point thereof with the bigger pressure value of the pressure sensors to be the force point of head, and determining the force point thereof with the smaller pressure value of the pressure sensors to be the force point of feet; and determining the force point which is of the largest pressure value of the pressure sensor to be the force point of hip;
if n=5, finding two critical force points which are of the longest distance from each other and where the pressure values of the sensors are not zero in the length of the mattress; determining the force point thereof with the bigger pressure value of the pressure sensors to be the force point of head, and determining the force point thereof with the smaller pressure value of the pressure sensors to be the force point of feet; determining the force point which is of the largest pressure value of the pressure sensor to be the force point of hip; finding two critical force points which are of the longest distance from each other where the pressure values of the sensors are not zero in the width of the mattress, and determining the two critical force points to be the force points of hands.

In one embodiment, if n=2 and the n critical force points are the force point of head and the force point of feet, the second determining module is further configured to: determine the ratio of leg to body for the user according to his information prestored in the terminal; determine the force point of hip according to the ratio of leg to body and the positions of the force point of head and the force point of feet; determine a predefined area of both sides of the distance from the force point of head to the force point of hip to be the force ranges of the arms; and determine the force point of head, the force points of arms, the force point of hip and the force point of feet to be all the critical force points for the user on the mattress.

In one embodiment, if n=3 and the n critical force points are the force point of head, the force point of feet and the force point of hip, the second determining module is further configured to: determine a predefined area of both sides of the distance from the force point of head to the force point of hip to be the force ranges of the arms; and determine the force point of head, the force points of arms, the force point of hip and the force point of feet to be all the critical force points for the user on the mattress.

In one embodiment, if n=5 and the n critical force points are the force point of head, the force point of feet, the force point of hip and the force points of hands, the second determining module is further configured to: determine the two ranges from the force points of hands to the force point of head to be the force points of arms; and determine the force point of head, the force points of arms, the force point of hip and the force point of feet to be all the critical force points for the user on the mattress.

In one embodiment, the information sending sub-module comprises: a first sending sub-module, configured to generating exercise information including the exercise status, and send the exercise information to the terminal , which determines the training parts of body of the user according to the exercise status; or a second sending sub-module, configured to acquire the number of the movement, generate the exercise information including the exercise status and the number of movement , and send the exercise information to the terminal , which computes total energy the user costs on the mattress according to the number of movement and the energy consumption of a single movement.

According to a third aspect, the invention relates to a device for exercise status recording, which applies to a mattress that has pressure sensors distributed thereon and is connected to a terminal wirelessly, the device comprising: processor; memory, used to store processor-executable instructions; wherein, the processor is configured to implement the method of Claim 1.

The technical solution provided in the embodiments of the disclosure can have the following advantageous:
by determining critical force points for a user on the mattress; determining the exercise status if the user according to the variation of the pressure values of the pressure sensors at the critical force points; and sending the exercise information including the exercise status to the terminal . So the physical exercise the user is doing can be sent to the terminal. Thanks to recording, by the terminal, the physical exercise the user is doing, the problem of electrical devices not being able to record other exercises except walking can be solved, and the effect that enriching exercises a user can do is achieved

It is to be understood that both the foregoing general description and the following detailed description are exemplary only and are not restrictive of the disclosure.

### BRIEF DESCRIPTION OF THE DRAWINGS

The accompanying drawings, which are incorporated in and constitute a part of this specification, illustrate embodiments consistent with the invention and, together with the description, serve to explain the principles of the invention.
FIG. 1 is a flow chart showing a method for exercise recording according to an exemplary embodiment.
FIG. 2A is a flow chart showing a method for exercise recording according to another exemplary embodiment.
FIG. 2B is a schematic diagram showing sit-up according to another exemplary embodiment.
Fig. 2C is a schematic diagram showing lying and leg lift according to another exemplary embodiment.
Fig. 2D is a schematic diagram showing pushup according to another exemplary embodiment.
Fig. 3 is a block diagram illustrating a device for exercise recording according to an exemplary embodiment.
Fig. 4 is a block diagram illustrating a device for exercise recording according to an exemplary embodiment.

### DETAILED DESCRIPTION

Reference will now be made in detail to exemplary embodiments, examples of which are illustrated in the accompanying drawings. The following description refers to the accompanying drawings in which same numbers in different drawings represent same or similar elements unless otherwise described. The implementations set forth in the following description of exemplary embodiments do not represent all implementations consistent with the invention. Instead, they are merely examples of devices and methods consistent with aspects related to the invention as recited in the appended claims.

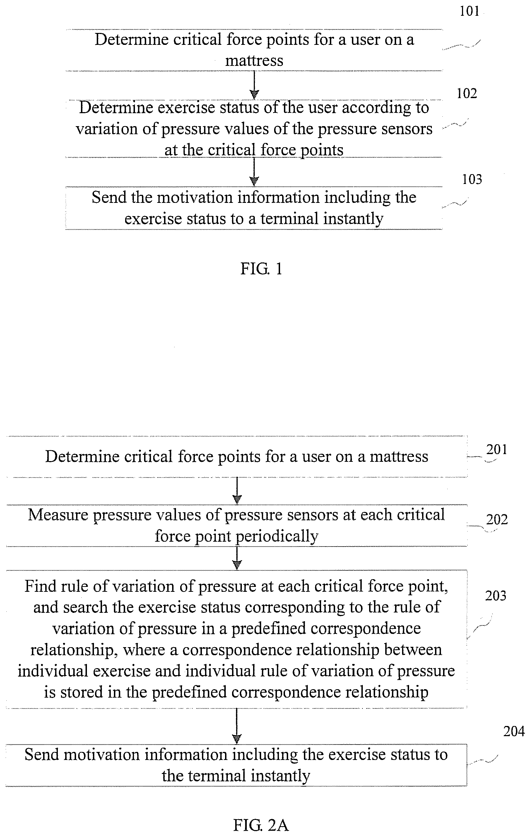

FIG. 1 is a flow chart showing a method for exercise recording according to an exemplary embodiment. The method for exercise recording applies to a mattress, which has pressure sensors distributed thereon and is connected to a terminal wirelessly. As shown in FIG. 1, the method includes the following steps.

In step 101, determine critical force points for a user on the mattress.

In step 102, determine the exercise status of the user on the mattress according to the variation of the pressure values of the pressure sensors at the critical force points.

In step 103, send the exercise information including the exercise status to the terminal.
In summary, in the method provided in the disclosure, by determining critical force points for a user on the mattress; determining the exercise status of the user according to the variation of the pressure values of the pressure sensors at the critical force points; and sending the exercise information including the exercise status to the terminal. So the physical exercise status the user is doing can be sent to the terminal. Thanks to recording, by the terminal, the physical exercise status the user is doing, the problem of electrical devices not being able to cannot record other exercises except walking can be solved, and the effect that enriching exercises a user can do is enriched.

FIG. 2A is a flow chart showing a method for exercise recording according to another exemplary embodiment. The method for exercise recording applies to a mattress, which has pressure sensors distributed thereon and is connected to a terminal wirelessly. As shown in FIG. 2A, the method includes the following steps.

In step 101, determine critical force points for a user on the mattress.

The critical force points are the points for the body of the user to touch the mattress when the user is doing physical exercises. For example, the critical force points may be at least one of the critical force point of head, critical force point of feet, critical force point of hip and critical force points of arms.

When the mattress is triggered to determine the critical force points, a first way is to arrange a triggering button on the mattress, and if the user presses the triggering button, the mattress will be triggered to determine the critical force points; in a second way, a triggering button is arranged on a terminal, and if the user presses the triggering button, a triggering signal is sent to the mattress by the terminal, and the mattress is triggered to determine the critical force points by the triggering signal; in a third way, after the user lies down on the mattress, detect whether the user remains still for a period by the pressure of the pressure sensors, and if the user remains still for a period, the mattress is triggered to determine the critical force points.

Since different critical force points correspond to different exercises, the mattress may determine the critical force points according to the supported exercises. For example, if the exercise status is sit-up, the critical force points are the force point of head and the force point of feet, and the mattress only needs to determine these two critical force points for the moment; if the exercise status is pushup, the critical force points is the force points of arms and feet, and the mattress only needs to determine these two critical force points for the moment.

Since the more there are critical force points, the more accurate for the mattress to determine the exercise status. Therefore, in the embodiment, whatever the exercise status is, and no matter whether the exercise status is corresponding to all the critical force points, the mattress determines all the critical force points, and determines the exercise status according to all the critical force points. Wherein, all the critical force points are the force point of head, the force point of feet, the force point of hip and the force points of arms.

In the embodiment, the mattress may determine all the critical force points according to the pressure values of the pressure sensors, or may determine some critical force points according to the pressure values of the pressure sensors at first, and then determine all the critical force points according to the some critical force points. For the moment, the determining the critical force points for the user on the mattress may include: when the user is lying on the mattress, determining n critical force points for the user on the mattress according to the pressure values of the pressure sensors, wherein n≥2; determine all the critical force points for the user on the mattress according to the n critical force points.

In the embodiment, the process to determine n critical force points and all the critical force points is provided, wherein n=2, 3 or 5, and the determining process is as below.
1) If n=2, find two critical force points which are of the longest distance from each other and where the pressure values of the sensors are not zero in the length of the mattress; determine the force point thereof with the bigger pressure value of the pressure sensors to be the force point of head, and determine the force point thereof with the smaller pressure value of the pressure sensors to be the force point of feet.

Since head is the upperest position of the body, and the feet is the bottom position of the body, when a user is lying on the mattress, the two force points which are of the longest distance from each other and where the pressure values of the sensors are not zero in the length of the mattress may be determined to be the critical force point of head and the critical force point of feet. Furthermore, when the user is lying on the mattress, the pressure of head is bigger than that of the feet, so the mattress may compare the two pressure values of the two force points, and determine the force point thereof with the bigger value to be the force point of head, and determine the force point thereof with the smaller pressure to be the force point of feet.

After the force point of head and the force point of feet are determined, the determining all the critical force points for the user on the mattress according to the n critical force points including:
determining the ratio of leg to body for the user according to his information prestored in the terminal;
determining the force point of hip according to the ratio of leg to body and the positions of the force point of head and the force point of feet;
determining a predefined area of both sides of the distance from the force point of head to the force point of hip to be the force ranges of the arms;
determining the force point of head, the force points of arms, the force point of hip and the force point of feet to be all the critical force points for the user on the mattress.

A user may store his information in the terminal previously, such as the gender, height, weight, the length of leg, and the ratio of leg to body or the like. The terminal sends the information of the user to the mattress, which determines the force point of hip according to the length of leg or the ratio of leg to body after determining the force points of head and the force points of leg. Wherein, the ratio of leg to body is the height of perineum multiplied by 100 to the height of the body; the average of the ratio for Asian male is 45.70, the ratio of Asian female is 44.90%, the average of the ratio for western male is 47.68, the ratio of western female is 47.34.

For example, if the distance from the force point of head to the force point of feet is 1.6 meters, and the length of leg is 0.7 meter, the force point which is 0.7 meter away from the force point of hip in the length will be determined to be the force point of hip. In another example, if the distance from the force point of head to the force point of feet is 1.6 meters, and the ratio of leg to body is 44.90, the length of leg will be calculated according to the equation: the length of leg=44.9/100*1.6=0.7 meter, and the force point which is 0.7 meter away from the force point of hip in the length will be determined to be the force point of hip.

Since the arms are located at both sides of the upper body of the user, so a predefined area of the both sides of the distance from the force point of head to the force point of hip is determined to be the force range of arms, and the predefined area is larger than or equal to the length of the arm of the user. For example, if the predefined area is 1 meter, then the area of both sides of 1 meter from the force point of head to the force point of hip is determined to be the force ranges of the arms.
2) if n=3, find two critical force points which are of the longest distance from each other and where the pressure values of the sensors are not zero in the length of the mattress; determine the force point thereof with the bigger pressure value of the pressure sensor to be the force point of head, and determine the force point thereof with the smaller pressure value of the pressure sensor to be the force point of feet; and determine the force point which is of the largest pressure of the pressure sensors to be the force point of hip.

Since the pressure of hip will be the largest when the user is lying down the mattress, the force point with the largest pressure in the length is determined to be the force point of hip by the mattress. Additionally, the method for the mattress to determine the force point of head and the force point of feet is the same as that in 1), which is not elaborated herein.

After the force point of head, the force point of hip and the force point of feet are determined, the determining all the critical force points for the user on the mattress according to the n critical force points including:
determining a predefined area of the both sides of the distance from the force point of head to the force point of hip to be the force ranges of arms;
determining the force point of head, the force points of arms, the force point of hip and the force point of feet to be all the critical force points for the user on the mattress.

Wherein, the method for the mattress to determine the force points of arms is the same as that in 1), which is not elaborated herein.
3) if n=5, find two critical force points which are of the longest distance from each other and where the pressure values of the sensors are not zero in the length of the mattress; determine the force point thereof with the bigger pressure value of the pressure sensors to be the force point of head, and determine the force point thereof with the smaller pressure value of the pressure sensors to be the force point of feet; determine the force point with the largest pressure value of the pressure sensor to be the force point of hip; find two force points which are of the longest distance from each other where the pressure values of the sensors are not zero in the width of the mattress, and determine the two force points to be the force points of hands.

The pressure values of the force points of the two hands in the width of the mattress are the same. The way for the mattress to determine the force point of head, the force point of hip and the force point of feet is the same as that in 1), which it is not elaborated herein.

After the force point of head, the force point of hip, the force point of feet and the force points of hands are determined, the determining all the critical force points for the user on the mattress according to the n critical force points including:
determining a predefined area from the two force points of hands to the force point of head to be the force points of arms;
determining the force point of head, the force points of arms, the force point of hip and the force point of feet to be all the critical force points for the user on the mattress.

The mattress may determine the area between the force point of head to the force points of each hand to be the force point of arm.

In step 202, measure the pressure values of the pressure sensors at each critical force point periodically.

In step 203, find the rule of variation of the pressure at individual critical force point, and searching the exercise status corresponding to the rule of variation of the pressure in a predefined correspondence relationship, where the correspondence relationship of individual exercise status to individual rule of variation of the pressure is stored in the predefined correspondence relationship.

The predefined correspondence relationship is used to store the correspondence relationship between exercise status and rule of variation of pressure. The rule of variation of pressure for several common exercises will be analyzed below.

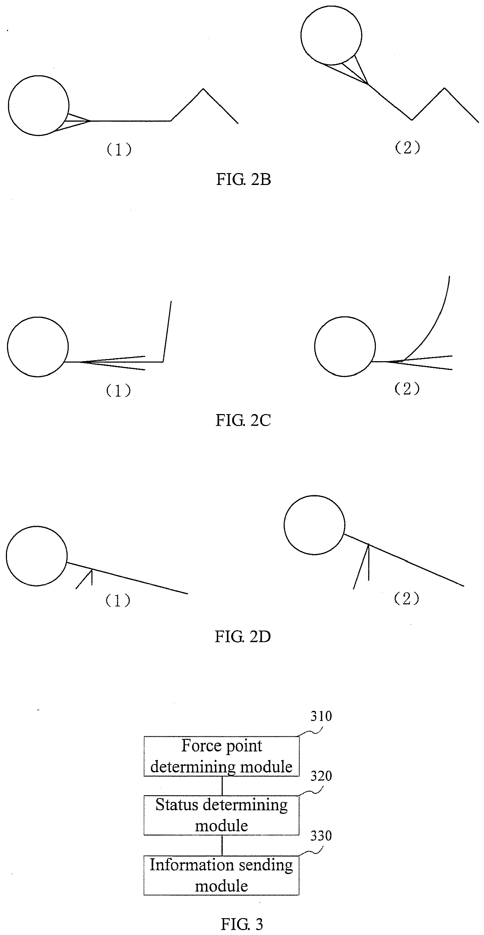

If the exercise status is sit-up, refer to the schematic diagram as shown in Fig. 2B. If the user is in the status 1 of lying, the pressure values of the force point of head, the force point of hip and the force point of feet are not changed, and the pressure values of the force points of arms are zero; if people is in status 2 of sit-up, the pressure value of the critical force point of feet is not changed, the pressure value of hip increases, and the pressure values of the force points of arms and the force point of head are zero. The mattress stores the correspondence relationships between the rule of variation of pressure and sit-up in the predefined correspondence relationship.

If the exercise status is lying and leg lift, refer to the schematic diagram about lying and leg lift as shown in Fig. 2C. If the user is in the status 1 of lying the pressure values of the force points of arms, the force point of head and the force point of hip are not changed, and the pressure value of the force point of feet is zero; if people is in the status 2 of leg lift, the pressure values of the force points of arms and the force point of head are not changed, and the pressure values of the force point of hip and the force point of feet are zero. The mattress stores the correspondence relationships between the rule of variation of pressure and sit-up in the predefined correspondence relationship.

If the exercise status is pushup, refer to the schematic diagram about pushup as shown in Fig. 2D. If the user is in the status 1 of prone, the pressure values of the force points of arms and the force point of feet are not changed, and the pressure values of the force point of head and the force point of hip are zero; if people is in the status 2 of propping up, the pressure values of the critical force points of arms and feet increase, and the pressure values of the force point of head and the force point of hip are zero. The mattress stores the correspondence relationships between the rule of variation of pressure and pushup in the predefined correspondence relationship.

In step 204, the exercise information including the exercise status is sent to the terminal.

In the embodiment, if the mattress includes a display, the exercise information may also be displayed in the display of the mattress, and the user may define his movement according to the displaying contents; if a display is not included in the mattress, the mattress may also send the exercise information including exercise status to the terminal, which records the movement of the user according to the exercise information, and the user may check his movement.

Two transmission modes are provided in the embodiment, and the two transmission modes are introduced respectively below.

In the first transmission mode, the exercise information including exercise status is generated, and sent to the terminal, which determines the training parts of body of the user according to the exercise status.

Wherein, the correspondence relationship between exercise status and training parts of the body is previously stored in the terminal; after the exercise status sent by the mattress is received, find the corresponding training part in the correspondence relationship, and provide it to the user. For example, the training part corresponding to sit-up is the stomach, and the training part of body corresponding to pushup is breast, etc.

In the second transmission mode, acquire the number of movement, generate the exercise information including the exercise status and the number, and send the exercise information to the terminal, which calculates total energy consumption for the user on the mattress according to the number and the energy consumption of a single movement.

The mattress may also determine the number of movement according to the rule of variation of pressure, and send the exercise information including the exercise status and the number to the terminal, which queries the calories a single movement costs, and multiply the number by the calories a single movement costs to obtain the total calories consumption for the user in the exercise status.

Of course, in addition to determining the training parts of body and calories according to the information of the user, the terminal may also perform other calculations according to the exercise information, and the embodiment is not intended to limit the use of exercise information.

FIG. 3 is a block diagram of a method for exercise recording according to an exemplary embodiment. The device for exercise recording applies to a mattress, which has pressure sensors distributed thereon and is connected to a terminal wirelessly. As shown in FIG. 3, the device for exercise recording includes force point determining module 310, status determining module 320 and information sending module 330.

The force point determining module 310, is configured to determine critical force points for a user on the mattress.

The status determining module 320, is configured to determine the exercise status of the user according to the variation of the pressure values of the pressure sensors at the critical force points which are determined by the force point determining module 310.

The information sending module 330, is configured to send the exercise information including the exercise status determined by the status determining module 320 to the terminal.

In summary, in the method provided in the disclosure, by determining critical force points for a user on the mattress; determining the exercise status of the user according to the variation of the pressure values of the pressure sensors at the critical force points; and sending the exercise information including the exercise status to the terminal. So the physical exercise the user is doing can be sent to the terminal. Thanks to recording, by the terminal, the physical exercise the user is doing, the problem of electrical devices not being able to record other exercises except walking can be solved, and the effect that enriching exercises a user can do is achieved.

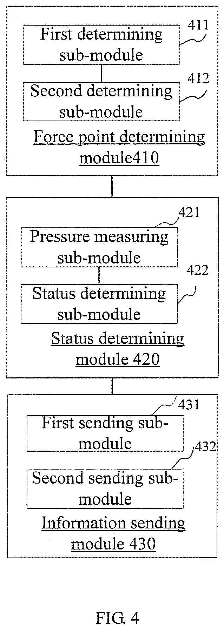

FIG. 4 is a block diagram of a method for exercise recording according to an exemplary embodiment. The device for exercise recording applies to a mattress, which has pressure sensors distributed thereon and is connected to a terminal wirelessly. As shown in FIG. 4, the device for exercise recording includes force point determining module 410, status determining module 420 and information sending module 430.

The force point determining module 410, is configured to determine critical force points for a user on the mattress.

The status determining module 420, is configured to determine the exercise status of the user according to the variation of the pressure values of the pressure sensors at the critical force points which is determined by the force point determining module 310.

The information sending module 430, is configured to send the exercise information including the exercise status determined by the status determining module 320 to the terminal.

In one embodiment, the force point determining module 410 includes a first determining sub-module 411 and a second determining sub-module 412;
the first determining sub-module 411, is configured to determine the n critical force points for the user on the mattress according to the pressure value of the pressure sensors when the user is lying on the mattress, wherein n≥2;
the second determining sub-module 412, is configured to determine all the critical force points for the user on the mattress according to the n critical force points determined by the first determining sub-module 411.

In one embodiment, the first determining sub-module 411 is further configured to:
if n=2, find two critical force points which are of the longest distance from each other and where the pressure values of the sensors are not zero in the length of the mattress; determine the force point thereof with the bigger pressure value of the pressure sensors to be the force point of head, and determine the force point thereof with the smaller pressure value of the pressure sensors to be the force point of feet.
if n=3, find two critical force points which are of the longest distance from each other and where the pressure values of the sensors are not zero in the length of the mattress; determine the force point thereof with the bigger pressure value of the pressure sensor to be the force point of head, and determine the force point thereof with the smaller pressure value of the pressure sensor to be the force point of feet; and determine the force point which is of the largest pressure of the pressure sensors to be the force point of hip.
if n=5, find two critical force points which are of the longest distance from each other and where the pressure values of the sensors are not zero in the length of the mattress; determine the force point thereof with the bigger pressure value of the pressure sensors to be the force point of head, and determine the force point thereof with the smaller pressure value of the pressure sensors to be the force point of feet; determine the force point with the largest pressure value of the pressure sensor to be the force point of hip; find two force points which are of the longest distance from each other where the pressure values of the sensors are not zero in the width of the mattress, and determine the two force points to be the force points of hands.

In one embodiment, if n=2 and the n key force points are the force point of head and the force point of feet, the second determining sub-module 412 is further configured to:
determine the ratio of leg to body for the user according to his information prestored in the terminal;
determine the force point of hip according to the ratio of leg to body and the positions of the force point of head and the force point of feet;
determine a predefined area of both sides of the distance from the force point of head to the force point of hip to be the force ranges of the arms; and
determine the force point of head, the force points of arms, the force point of hip and the force point of feet to be all the critical force points for the user on the mattress.

In one embodiment, if n=3 and the n key force points are the force point of head, the force point of feet and the force point of hip, the second determining sub-module 412 is further configured to:
determine a predefined area of both sides of the distance from the force point of head to the force point of hip to be the force ranges of the arms; and
determine the force point of head, the force points of arms, the force point of hip and the force point of feet to be all the critical force points for the user on the mattress.

In one embodiment, if n=5 and the n key force points are the force point of head, the force point of feet, the force point of hip and the force points of hands, the second determining sub-module 412 is further configured to:
determine the two ranges from the force points of hands to the force point of head to be the force points of arms; and
determine the force point of head, the force points of arms, the force point of hip and the force point of feet to be all the critical force points for the user on the mattress.

In one embodiment, the status determining sub-module 420 includes pressure measuring sub-module 421 and status determining sub-module 422;

The pressure measuring sub-module 421 is configured to measure the pressure of the pressure sensors at each critical force point periodically;
status determining sub-module 422, configured to find the rule of variation of the pressure at each critical force point, and search the exercise status corresponding to the rule of variation of the pressure in a predefined correspondence relationship, where a correspondence relationship between individual exercise status and individual rule of variation of the pressure is stored in the predefined correspondence relationship.

In one embodiment, the information sending module 430 includes a first sending sub-module 431 and a second sending sub-module 432;
the first sending sub-module 431, is configured to generating exercise information including the exercise status, and send the exercise information to the terminal , which determines the training parts of body of the user according to the exercise status; or
the second sending sub-module 432, is configured to acquire the number of the movement, generate the exercise information including the exercise status and the number of movement , and send the exercise information to the terminal, which computes total energy the user costs on the mattress according to the number of movement and the energy consumption of a single movement.

In summary, in the method provided in the disclosure, by determining critical force points for a user on the mattress; determining the exercise status of the user according to the variation of the pressure values of the pressure sensors at the critical force points; and sending the exercise information including the exercise status to the terminal. So the physical exercise the user is doing can be sent to the terminal. Thanks to recording, by the terminal, the physical exercise the user is doing, the problem of electrical devices not being able to record other exercises except walking can be solved, and the effect that enriching exercises a user can do is achieved.

Additionally, by determining the force point of hip according to the ratio of leg to body, the force point of head and the force point of feet of the user, the position of the force point of hip of the user may be calculated accurately according to the ratio of leg to body of the user, and thus the accuracy of the critical force points is improved.

Additionally, by sending the exercise information including exercise status to the terminal, the terminal may mark the training part of the body of the user according to the exercise information, calculate the energy consumption or the like, so that a more comprehensive exercise recording may be provided to the user.

With respect to the devices in the above embodiments, the specific manners for performing operations for individual modules and sub-modules therein have been described in details in the embodiments regarding the methods, which will not be elaborated herein.

A device for exercise recording is provided in the embodiment of the present disclosure, which is able to implement the method for exercise recording provided in the disclosure, and the device applies to a mattress that has pressure sensors distributed thereon and is connected to a terminal wirelessly, the device including: processor; memory, used to store processor-executable instructions;
wherein, the processor is configured to:
determine critical force points for a user on the mattress;
determine the exercise status of the user on the mattress according to the variation of the pressure values of the pressure sensors at the critical force points; and
send the exercise information including the exercise status to the terminal.

Other embodiments of the invention will be apparent to those skilled in the art from consideration of the specification and practice of the disclosures herein. This application is intended to cover any variations, uses, or adaptations of the disclosure following the general principles thereof and including such departures from the present disclosure as come within known or customary practice in the art. It is intended that the specification and examples be considered as exemplary only, with a true scope of the invention being indicated by the following claims.

It is intended that the scope of the invention only be limited by the appended claims.

## Claims

1. A method for exercise recording, which applies to a mattress that has pressure sensors thereon and is connected to a terminal wirelessly, comprising:
determining (101) critical force points for a user on the mattress, wherein said critical force points are the points for the body of the user to touch the mattress when the user is doing physical exercises;
determining (102) exercise status of the user on the mattress according to the difference of pressure values of the pressure sensors at different critical force points; and
sending (103) exercise information to a terminal, the exercise information including the exercise status, said method being **characterized in that** said determining the exercise status of the user on the mattress according to the variation of the pressure values of the pressure sensors at the critical force points comprises:
measuring (202) the pressure values of the pressure sensors at each critical force point periodically;
finding (203) rule of variation of the pressure at each critical force point, and searching the exercise status corresponding to the rule of variation of the pressure in a predefined correspondence relationship which is stored in the mattress, where a correspondence relationship between individual exercise status and individual rules of variation of the pressure is stored in the predefined correspondence relationship, wherein:
a/ if the variation of pressure is determined as follows:
the pressure value of the force point of head changes from an unchanged pressure to zero,
the pressure value of the force point of hip increases,
the pressure value of the critical force point of feet remains unchanged, and
the pressure values of the force points of arms remains zero, the exercise status is determined as sitting-up from a lying position;
b/ if the variation of pressure is determined as follows:
the pressure values of the force points of arms remains unchanged,
the pressure values of the force points head remains unchanged,
the pressure values of the force point of hip changes from unchanged to zero, and
the pressure value of the force point of feet remains zero, the exercise status is determined as lifting leg from a lying position;
c/ if the variation of pressure is determined as follows:
the pressure values of the force point of head remains zero,
the pressure values of the force point of hip remains zero,
the pressure values of the force points of arms is increased and
the pressure value of the force point of feet is increased. the exercise status is determined as propping up from a pushup prone position.

2. The method of claim 1, **characterized in that** said determining critical force points for a user on the mattress comprises:
determining n critical force points for the user on the mattress according to the pressure values of the pressure sensors when the user is lying on the mattress, wherein n≥2;
determining all the critical force points for the user on the mattress according to the n critical force points.

3. The method of claim 2, **characterized in that** said determining n critical force points for the user on the mattress according to the pressure values of the pressure sensors comprises:
if n=2, finding, in the length of the mattress, two critical force points which are of the longest distance from each other and where the pressure values of the sensors are not zero; and determining the force point thereof with the bigger pressure value of the pressure sensors to be the force point of head, and determining the force point thereof with the smaller pressure value of the pressure sensors to be the force point of feet;
if n=3, finding, in the length of the mattress, two critical force points which are of the longest distance from each other and where the pressure values of the sensors are not zero; determining the force point thereof with the bigger pressure value of the pressure sensors to be the force point of head, and determining the force point thereof with the smaller pressure value of the pressure sensors to be the force point of feet; and determining a force point which is of the largest pressure value of the pressure sensor to be the force point of hip;
if n=5, finding, in the length of the mattress, two critical force points which are of the longest distance from each other and where the pressure values of the sensors are not zero; determining the force point thereof with the bigger pressure value of the pressure sensors to be the force point of head, and determining the force point thereof with the smaller pressure value of the pressure sensors to be the force point of feet; determining a force point which is of the largest pressure value of the pressure sensor to be the force point of hip; finding, in the width of the mattress, two critical force points which are of the longest distance from each other and where the pressure values of the sensors are not zero, and determining the two critical force points to be two force points of hands.

4. The method of claim 3, **characterized in that** if n=2 and the n critical force points are the force point of head and the force point of feet, said determining all the critical force points for the user on the mattress according to the n critical force points comprises:
determining ratio of leg to body for the user according to his information prestored in the terminal;
determining the force point of hip according to the ratio of leg to body and the positions of the force point of head and the force point of feet;
determining a predefined area of both sides of the distance from the force point of head to the force point of hip to be force ranges of the arms; and
determining the force point of head, the force points of arms, the force point of hip and the force point of feet to be all the critical force points for the user on the mattress.

5. The method of claim 3, **characterized in that** if n=3 and the n critical force points are the force point of head, the force point of feet and the force point of hip, said determining all the critical force points for the user on the mattress according to the n critical force points comprises:
determining a predefined area of both sides of the distance from the force point of head to the force point of hip to be the force ranges of the arms; and
determining the force point of head, the force points of arms, the force point of hip and the force point of feet to be all the critical force points for the user on the mattress.

6. The method of claim 3, **characterized in that** if n=5 and the n critical force points are the force point of head, the force point of feet, the force point of hip and the force point of hands, said determining all the critical force points for the user on the mattress according to the n critical force points comprises:
determining respective ranges from the two force points of hands to the force point of head to be the force points of arms; and
determining the force point of head, the force points of arms, the force point of hip and the force point of feet to be all the critical force points for the user on the mattress.

7. The method of claim 1, **characterized in that** said sending the exercise information including the exercise status to the terminal comprises:
generating exercise information including the exercise status , and sending the exercise information to the terminal, which determines the training parts of body of the user according to the exercise status; or
acquiring the number of the movement of the exercise status, generating the exercise information including the exercise status and the number of movement , and sending the exercise information to the terminal, which computes total energy the user costs on the mattress according to the number of movement and the energy consumption of a single movement.

8. A device for exercise recording, which applies to a mattress that has pressure sensors distributed thereon and is connected to a terminal wirelessly, the device comprising:
a force point determining module (310), configured to determine critical force points for a user on the mattress, wherein said critical force points are the points for the body of the user to touch the mattress when the user is doing physical exercises;
a status determining module (320), configured to determine exercise status of the user on the mattress according to difference of pressure values of the pressure sensors at different critical force points; and
an information sending module (330), configured to send the exercise information to a terminal, the exercise information including the exercise status, said device being **characterized in that** the status determining module comprises:
a pressure measuring sub-module (421), configured to measure the pressure values of the pressure sensors at each critical force point periodically; and
a status determining sub-moduie (422), configured to find rule of variation of pressure at each critical force point, and search the exercise status corresponding to the rule of variation of pressure in a predefined correspondence relationship which is stored in the mattress, where a correspondence relationship between individual exercise status and individual rule of variation of pressure is stored in the predefined correspondence relationship,
wherein:
a/ if the variation of pressure is determined as follows:
the pressure value of the force point of head changes from an unchanged pressure to zero,
the pressure value of the force point of hip increases,
the pressure value of the critical force point of feet remains unchanged, and the pressure values of the force points of arms remains zero,
the exercise status is determined as sitting-up from a lying position;
b/ if the variation of pressure is determined as follows:
the pressure values of the force points of arms remains unchanged,
the pressure values of the force points head remains unchanged,
the pressure values of the force point of hip changes from unchanged to zero, and
the pressure value of the force point of feet remains zero,
the exercise status is determined as lifting leg from a lying position;
c/ if the variation of pressure is determined as follows:
the pressure values of the force point of head remains zero,
the pressure values of the force point of hip remains zero,
the pressure values of the force points of arms is increased and
the pressure value of the force point of feet is increased,
the exercise status is determined as propping up from a pushup prone position.

9. The device of claim 8, **characterized in that** the force point determining module comprises:
a first determining sub-module (411), configured to determine n critical force points for the user on the mattress according to the pressure values of the pressure sensors when the user is lying on the mattress, wherein n≥2; and
a second determining sub-module (412), configured to determine all the critical force points for the user on the mattress according to the n critical force points determined by the first determining sub-module.

10. The device of claim 9, **characterized in that** the first determining sub-module is further configured to:
if n=2, finding, in the length of the mattress, two critical force points which are of the longest distance from each other and where the pressure values of the sensors are not zero; and determining the force point thereof with the bigger pressure value of the pressure sensors to be the force point of head, and determining the force point thereof with the smaller pressure value of the pressure sensors to be the force point of feet;
if n=3, finding, in the length of the mattress, two critical force points which are of the longest distance from each other and where the pressure values of the sensors are not zero; determining the force point thereof with the bigger pressure value of the pressure sensors to be the force point of head, and determining the force point thereof with the smaller pressure value of the pressure sensors to be the force point of feet; and determining a force point which is of the largest pressure value of the pressure sensor to be the force point of hip;
if n=5, finding, in the length of the mattress, two critical force points which are of the longest distance from each other and where the pressure values of the sensors are not zero; determining the force point thereof with the bigger pressure value of the pressure sensors to be the force point of head, and determining the force point thereof with the smaller pressure value of the pressure sensors to be the force point of feet; determining a force point which is of the largest pressure value of the pressure sensor to be the force point of hip; finding, in the width of the mattress, two critical force points which are of the longest distance from each other and where the pressure values of the sensors are not zero, and determining the two critical force points to be two force points of hands.

11. The device of claim 10, **characterized in that** if n=2 and the n critical force points are the force point of head and the force point of feet, the second determining module is further configured to:
determine ratio of leg to body for the user according to his information prestored in the terminal;
determine the force point of hip according to the ratio of leg to body and the positions of the force point of head and the force point of feet;
determine a predefined range of both sides of the distance from the force point of head to the force point of hip to be force ranges of the arms; and
determine the force point of head, the force points of arms, the force point of hip and the force point of feet to be all the critical force points for the user on the mattress.

12. The device of claim 8, **characterized in that** the information sending module comprises:
a first sending sub-module (431), configured to generate exercise information including the exercise status, and send the exercise information to the terminal, which determines the training parts of body of the user according to the exercise status; or
a second sending sub-module (432), configured to acquire the number of movement of the exercise status, generate the exercise information including the exercise status and the number of movement, and send the exercise information to the terminal, which computes total energy the user costs on the mattress according to the number of movement and energy consumption of a single movement.

## Patentansprüche

1. Verfahren zur Aufzeichnung von Übungen, das bei einer Matratze anwendbar ist, die Drucksensoren daran aufweist und drahtlos mit einem Endgerät verbunden ist, umfassend:
Bestimmen (101) entscheidender Kraftpunkte für einen Benutzer auf der Matratze, wobei die entscheidenden Kraftpunkte die Punkte sind, an denen der Körper des Benutzers die Matratze berührt, wenn der Benutzer körperliche Übungen durchführt,
Bestimmen (102) eines Übungsstatus des Benutzers auf der Matratze gemäß einer Differenz von Druckwerten der Drucksensoren an unterschiedlichen entscheidenden Kraftpunkten, und
Senden (103) von Übungsinformationen an ein Endgerät, wobei die Übungsinformationen den Übungsstatus beinhalten, wobei das Verfahren **dadurch gekennzeichnet ist, dass** das Bestimmen des Übungsstatus des Benutzers auf der Matratze gemäß der Veränderung der Druckwerte der Drucksensoren an den entscheidenden Kraftpunkten umfasst:
periodisches Messen (202) der Druckwerte der Drucksensoren an jedem entscheidenden Kraftpunkt,
Auffinden (203) einer Regel der Veränderung des Drucks an jedem entscheidenden Kraftpunkt und Suchen des Übungsstatus entsprechend der Regel der Veränderung des Drucks in einer vordefinierten Übereinstimmungsbeziehung, welche in der Matratze gespeichert wird, wobei eine Übereinstimmungsbeziehung zwischen einzelnen Übungsstatus und einzelnen Regeln der Veränderung des Drucks in der vordefinierten Übereinstimmungsbeziehung gespeichert wird, wobei:
a/ wenn die Druckveränderung wie folgt bestimmt wird:
der Druckwert des Kraftpunktes des Kopfes verändert sich von einem unveränderten Druck zu Null,
der Druckwert des Kraftpunktes der Hüfte erhöht sich,
der Druckwert des entscheidenden Kraftpunktes der Füße bleibt unverändert, und
die Druckwerte der Kraftpunkte der Arme bleiben Null,
wird der Übungsstatus als Aufrichten aus einer Liegeposition bestimmt,
b/ wenn die Druckveränderung wie folgt bestimmt wird:
die Druckwerte der Kraftpunkte der Arme bleiben unverändert,
die Druckwerte der Kraftpunkte des Kopfes bleiben unverändert,
die Druckwerte des Kraftpunktes der Hüfte verändern sich von unverändert zu Null, und
der Druckwert des Kraftpunktes der Füße bleibt Null,
wird der Übungsstatus als Anheben eines Beines aus einer Liegeposition bestimmt,
c/wenn die Druckveränderung wie folgt bestimmt wird:
die Druckwerte des Kraftpunktes des Kopfes bleiben Null,
die Druckwerte des Kraftpunktes der Hüfte bleiben Null,
die Druckwerte der Kraftpunkte der Arme werden erhöht, und
der Druckwert des Kraftpunktes der Füße wird erhöht,
wird der Übungsstatus als Abstützen aus einer Liegestütze-Bauchlage bestimmt.

2. Verfahren nach Anspruch 1, **dadurch gekennzeichnet, dass** das Bestimmen entscheidender Kraftpunkte für einen Benutzer auf der Matratze umfasst:
Bestimmen von n entscheidenden Kraftpunkten für den Benutzer auf der Matratze gemäß den Druckwerten der Drucksensoren, wenn der Benutzer auf der Matratze liegt, wobei n ≥ 2,
Bestimmen aller entscheidenden Kraftpunkte für den Benutzer auf der Matratze gemäß den n entscheidenden Kraftpunkten.

3. Verfahren nach Anspruch 2, **dadurch gekennzeichnet, dass** das Bestimmen von n entscheidenden Kraftpunkten für den Benutzer auf der Matratze gemäß den Druckwerten der Drucksensoren umfasst:
wenn n = 2, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist,
wenn n = 3, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist, und Bestimmen, dass ein Kraftpunkt, der den größten Druckwert der Drucksensoren aufweist, der Kraftpunkt der Hüfte ist,
wenn n = 5, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist, Bestimmen, dass ein Kraftpunkt, der den größten Druckwert der Drucksensoren aufweist, der Kraftpunkt der Hüfte ist, und Auffinden, entlang der Breite der Matratze, von zwei entscheidenden Kraftpunkten, die den größten Abstand zueinander aufweisen und an denen die Druckwerte der Sensoren nicht Null sind, und Bestimmen, dass die zwei entscheidenden Kraftpunkte die zwei Kraftpunkte der Hände sind.

4. Verfahren nach Anspruch 3, **dadurch gekennzeichnet, dass**, wenn n = 2 und die n entscheidenden Kraftpunkte der Kraftpunkt des Kopfes und der Kraftpunkt der Füße sind, das Bestimmen aller entscheidenden Kraftpunkte für den Benutzer auf der Matratze gemäß den n entscheidenden Kraftpunkten umfasst:
Bestimmen eines Bein-Körper-Verhältnisses für den Benutzer gemäß seinen in dem Endgerät vorgespeicherten Informationen,
Bestimmen des Kraftpunktes der Hüfte gemäß dem Bein-Körper-Verhältnis und den Positionen des Kraftpunktes des Kopfes und des Kraftpunktes der Füße,
Bestimmen, dass ein vordefinierter Bereich beider Seiten des Abstandes von dem Kraftpunkt des Kopfes zu dem Kraftpunkt der Hüfte Kraftbereiche der Arme sind, und
Bestimmen, dass der Kraftpunkt des Kopfes, die Kraftpunkte der Arme, der Kraftpunkt der Hüfte und der Kraftpunkt der Füße alle entscheidenden Kraftpunkte für den Benutzer auf der Matratze sind.

5. Verfahren nach Anspruch 3, **dadurch gekennzeichnet, dass**, wenn n = 3 und die n entscheidenden Kraftpunkte der Kraftpunkt des Kopfes, der Kraftpunkt der Füße und der Kraftpunkt der Hüfte sind, das Bestimmen aller entscheidenden Kraftpunkte für den Benutzer auf der Matratze gemäß den n entscheidenden Kraftpunkten umfasst:
Bestimmen, dass ein vordefinierter Bereich beider Seiten des Abstandes von dem Kraftpunkt des Kopfes zu dem Kraftpunkt der Hüfte die Kraftbereiche der Arme sind, und
Bestimmen, dass der Kraftpunkt des Kopfes, die Kraftpunkte der Arme, der Kraftpunkt der Hüfte und der Kraftpunkt der Füße alle entscheidenden Kraftpunkte für den Benutzer auf der Matratze sind.

6. Verfahren nach Anspruch 3, **dadurch gekennzeichnet, dass**, wenn n = 5 und die n entscheidenden Kraftpunkte der Kraftpunkt des Kopfes, der Kraftpunkt der Füße, der Kraftpunkt der Hüfte und der Kraftpunkt der Hände sind, das Bestimmen aller entscheidenden Kraftpunkte für den Benutzer auf der Matratze gemäß den n entscheidenden Kraftpunkten umfasst:
Bestimmen, dass jeweilige Bereiche von den zwei Kraftpunkten der Hände zu dem Kraftpunkt des Kopfes die Kraftpunkte der Arme sind, und
Bestimmen, dass der Kraftpunkt des Kopfes, die Kraftpunkte der Arme, der Kraftpunkt der Hüfte und der Kraftpunkt der Füße alle entscheidenden Kraftpunkte für den Benutzer auf der Matratze sind.

7. Verfahren nach Anspruch 1, **dadurch gekennzeichnet, dass** das Senden der Übungsinformationen, die den Übungsstatus beinhalten, an das Endgerät umfasst:
Erzeugen von Übungsinformationen, die den Übungsstatus beinhalten, und Senden der Übungsinformationen an das Endgerät, welches die Trainingskörperteile des Benutzers gemäß dem Übungsstatus bestimmt, oder
Erfassen der Bewegungsanzahl des Übungsstatus, Erzeugen der Übungsinformationen, die den Übungsstatus und die Bewegungsanzahl beinhalten, und Senden der Übungsinformationen an das Endgerät, welches eine Gesamtenergie, die der Benutzer auf der Matratze aufwendet, gemäß der Bewegungsanzahl und dem Energieverbrauch einer einzelnen Bewegung berechnet.

8. Vorrichtung zur Aufzeichnung von Übungen, die bei einer Matratze anwendbar ist, die daran verteilte Drucksensoren aufweist und drahtlos mit einem Endgerät verbunden ist, wobei die Vorrichtung umfasst:
ein Kraftpunktbestimmungsmodul (310), das dafür ausgelegt ist, entscheidende Kraftpunkte für einen Benutzer auf der Matratze zu bestimmen, wobei die entscheidenden Kraftpunkte die Punkte sind, an denen der Körper des Benutzers die Matratze berührt, wenn der Benutzer körperliche Übungen durchführt,
ein Statusbestimmungsmodul (320), das dafür ausgelegt ist, einen Übungsstatus des Benutzers auf der Matratze gemäß einer Differenz von Druckwerten der Drucksensoren an unterschiedlichen entscheidenden Kraftpunkten zu bestimmen, und
ein Informationssendemodul (330), das dafür ausgelegt ist, die Übungsinformationen an ein Endgerät zu senden, wobei die Übungsinformationen den Übungsstatus beinhalten, wobei die Vorrichtung **dadurch gekennzeichnet ist, dass** das Statusbestimmungsmodul umfasst:
ein Druckmessungsuntermodul (421), das dafür ausgelegt ist, die Druckwerte der Drucksensoren an jedem entscheidenden Kraftpunkt periodisch zu messen, und
ein Statusbestimmungsuntermodul (422), das dafür ausgelegt ist, eine Regel der Veränderung des Drucks an jedem entscheidenden Kraftpunkt aufzufinden und den Übungsstatus entsprechend der Regel der Veränderung des Drucks in einer vordefinierten Übereinstimmungsbeziehung, welche in der Matratze gespeichert wird, zu suchen, wobei eine Übereinstimmungsbeziehung zwischen einem einzelnen Übungsstatus und einer einzelnen Regel der Veränderung des Drucks in der vordefinierten Übereinstimmungsbeziehung gespeichert wird,
wobei:
a/ wenn die Druckveränderung wie folgt bestimmt wird:
der Druckwert des Kraftpunktes des Kopfes verändert sich von einem unveränderten Druck zu Null,
der Druckwert des Kraftpunktes der Hüfte erhöht sich,
der Druckwert des entscheidenden Kraftpunktes der Füße bleibt unverändert, und
die Druckwerte der Kraftpunkte der Arme bleiben Null,
wird der Übungsstatus als Aufrichten aus einer Liegeposition bestimmt,
b/ wenn die Druckveränderung wie folgt bestimmt wird:
die Druckwerte der Kraftpunkte der Arme bleiben unverändert,
die Druckwerte der Kraftpunkte des Kopfes bleiben unverändert,
die Druckwerte des Kraftpunktes der Hüfte verändern sich von unverändert zu Null, und
der Druckwert des Kraftpunktes der Füße bleibt Null,
wird der Übungsstatus als Anheben eines Beines aus einer Liegeposition bestimmt,
c/ wenn die Druckveränderung wie folgt bestimmt wird:
die Druckwerte des Kraftpunktes des Kopfes bleiben Null,
die Druckwerte des Kraftpunktes der Hüfte bleiben Null,
die Druckwerte der Kraftpunkte der Arme werden erhöht, und
der Druckwert des Kraftpunktes der Füße wird erhöht,
wird der Übungsstatus als Abstützen aus einer Liegestütze-Bauchlage bestimmt.

9. Vorrichtung nach Anspruch 8, **dadurch gekennzeichnet, dass** das Kraftpunktbestimmungsmodul umfasst:
ein erstes Bestimmungsuntermodul (411), das dafür ausgelegt ist, n entscheidenden Kraftpunkte für den Benutzer auf der Matratze gemäß den Druckwerten der Drucksensoren zu bestimmen, wenn der Benutzer auf der Matratze liegt, wobei n ≥ 2, und
ein zweites Bestimmungsuntermodul (412), das dafür ausgelegt ist, alle entscheidenden Kraftpunkte für den Benutzer auf der Matratze gemäß den durch das erste Bestimmungsuntermodul bestimmten n entscheidenden Kraftpunkten zu bestimmen.

10. Vorrichtung nach Anspruch 9, **dadurch gekennzeichnet, dass** das erste Bestimmungsuntermodul ferner dafür ausgelegt ist:
wenn n = 2, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist,
wenn n = 3, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist, und Bestimmen, dass ein Kraftpunkt, der den größten Druckwert der Drucksensoren aufweist, der Kraftpunkt der Hüfte ist,
wenn n = 5, Auffinden, entlang der Länge der Matratze, von zwei entscheidenden Kraftpunkten, die den größten Abstand zueinander aufweisen und an denen die Druckwerte der Sensoren nicht Null sind, und Bestimmen, dass der Kraftpunkt davon, der den größeren Druckwert der Drucksensoren aufweist, der Kraftpunkt des Kopfes ist, und Bestimmen, dass der Kraftpunkt davon, der den kleineren Druckwert der Drucksensoren aufweist, der Kraftpunkt der Füße ist, Bestimmen, dass ein Kraftpunkt, der den größten Druckwert der Drucksensoren aufweist, der Kraftpunkt der Hüfte ist, und Auffinden, entlang der Breite der Matratze, von zwei entscheidenden Kraftpunkten, die den größten Abstand zueinander aufweisen und an denen die Druckwerte der Sensoren nicht Null sind, und Bestimmen, dass die zwei entscheidenden Kraftpunkte die zwei Kraftpunkte der Hände sind.

11. Vorrichtung nach Anspruch 10, **dadurch gekennzeichnet, dass**, wenn n = 2 und die n entscheidenden Kraftpunkte der Kraftpunkt des Kopfes und der Kraftpunkt der Füße sind, das zweite Bestimmungsmodul ferner dafür ausgelegt ist:
ein Bein-Körper-Verhältnis für den Benutzer gemäß seinen in dem Endgerät vorgespeicherten Informationen zu bestimmen,
den Kraftpunkt der Hüfte gemäß dem Bein-Körper-Verhältnis und den Positionen des Kraftpunktes des Kopfes und des Kraftpunktes der Füße zu bestimmen,
zu bestimmen, dass ein vordefinierter Bereich beider Seiten des Abstandes von dem Kraftpunkt des Kopfes zu dem Kraftpunkt der Hüfte Kraftbereiche der Arme sind, und
zu bestimmen, dass der Kraftpunkt des Kopfes, die Kraftpunkte der Arme, der Kraftpunkt der Hüfte und der Kraftpunkt der Füße alle entscheidenden Kraftpunkte für den Benutzer auf der Matratze sind.

12. Vorrichtung nach Anspruch 8, **dadurch gekennzeichnet, dass** das Informationssendemodul umfasst:
ein erstes Sendeuntermodul (431), das dafür ausgelegt ist, Übungsinformationen, die den Übungsstatus beinhalten, zu erzeugen und die Übungsinformationen an das Endgerät zu senden, welches die Trainingskörperteile des Benutzers gemäß dem Übungsstatus bestimmt, oder
ein zweites Sendeuntermodul (432), das dafür ausgelegt ist, die Bewegungsanzahl des Übungsstatus zu erfassen, die Übungsinformationen, die den Übungsstatus und die Bewegungsanzahl beinhalten, zu erzeugen und die Übungsinformationen an das Endgerät zu senden, welches eine Gesamtenergie, die der Benutzer auf der Matratze aufwendet, gemäß der Bewegungsanzahl und dem Energieverbrauch einer einzelnen Bewegung berechnet.

## Revendications

1. Procédé pour un enregistrement d'exercices, qui s'applique à un matelas qui présente des capteurs de pression et est relié à une borne sans fil, comprenant :
la détermination (101) de points de force critiques pour un utilisateur sur le matelas, dans lequel lesdits points de force critiques sont les points du corps de l'utilisateur qui touchent le matelas lorsque l'utilisateur réalise des exercices physiques ;
la détermination (102) d'un statut d'exercice de l'utilisateur sur le matelas en fonction de la différence de valeurs de pression des capteurs de pression en différents points de force critiques ; et
l'envoi (103) d'une information d'exercice à une borne, l'information d'exercice incluant le statut d'exercice, ledit procédé étant **caractérisé en ce que** ladite détermination du statut d'exercice de l'utilisateur sur le matelas en fonction de la variation des valeurs de pression des capteurs de pression au niveau des points de force critiques comprend :
la mesure (202) périodique de valeurs de pression des capteurs au niveau de chaque point de force critique ;
le fait de trouver (203) une règle de variation de la pression en chaque point de force critique, et la recherche du statut d'exercice correspondant à la règle de variation de la pression dans une relation de correspondance prédéfinie qui est stockée dans le matelas, où une relation de correspondance entre un statut d'exercice individuel et des règles de variation individuelles de la pression est stockée dans la relation de correspondance prédéfinie, dans lequel :
a/ si la variation de pression est déterminée comme suit :
la valeur de pression du point de force de tête passe d'une pression inchangée à zéro,
la valeur de pression du point de force de hanche augmente,
la valeur de pression du point de force critique de pieds demeure inchangée, et
les valeurs de pression des points de force de bras demeurent à zéro,
le statut d'exercice est déterminé comme étant le fait de s'asseoir depuis une position allongée ;
b/ si la variation de pression est déterminée comme suit :
les valeurs de pression des points de force de bras demeurent inchangées,
les valeurs de pression des points de force de tête demeurent inchangées,
les valeurs de pression du point de force de hanche passent d'inchangées à zéro, et
la valeur de pression du point de force de pieds demeure à zéro,
le statut d'exercice est déterminé comme étant le fait de soulever une jambe depuis une position allongée ;
c/ si la variation de pression est déterminée comme suit :
les valeurs de pression du point de force de tête demeurent à zéro,
les valeurs de pression du point de force de hanche demeurent à zéro,
les valeurs de pression des points de force de bras sont augmentées, et
la valeur de pression du point de force de pieds est augmentée,
le statut d'exercice est déterminé comme étant le fait de s'appuyer depuis une position prédisposée pour faire des pompes.

2. Procédé selon la revendication 1, **caractérisé en ce que** ladite détermination de points de force critiques pour un utilisateur sur le matelas comprend :
la détermination de n points de force critiques pour l'utilisateur sur le matelas en fonction des valeurs de pression des capteurs de pression lorsque l'utilisateur est allongé sur le matelas, dans lequel n ≥ 2 ;
la détermination de tous les points de force critiques pour l'utilisateur sur le matelas en fonction des n points de force critiques.

3. Procédé selon la revendication 2, **caractérisé en ce que** ladite détermination de n points de force critiques pour l'utilisateur sur le matelas en fonction des valeurs de pression des capteurs de pression comprend :
si n = 2, le fait de trouver, dans la longueur du matelas, deux points de force critiques qui sont de la distance la plus longue de l'un à l'autre et où les valeurs de pression des capteurs ne sont pas zéro ; et la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ;
si n = 3, le fait de trouver, dans la longueur du matelas, deux points de force critiques qui sont de la distance la plus longue de l'un à l'autre et où les valeurs de pression des capteurs ne sont pas zéro ; la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ; et la détermination d'un point de force qui est de la valeur de pression la plus grande du capteur de pression comme étant le point de force de hanche ;
si n = 5, le fait de trouver, dans la longueur du matelas, deux points de force critiques qui sont de la distance la plus longue de l'un à l'autre et où les valeurs de pression des capteurs ne sont pas zéro ; la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ; la détermination d'un point de force qui est de la valeur de pression la plus grande du capteur de pression comme étant le point de force de hanche ; le fait de trouver, dans la largeur du matelas, deux points de force critiques qui sont de la distance la plus longue de l'un à l'autre et où les valeurs de pression des capteurs ne sont pas zéro, et la détermination des deux points de force critiques comme étant deux points de force de mains.

4. Procédé selon la revendication 3, **caractérisé en ce que** si n = 2 et les n points de force critiques sont le point de force de tête et le point de force de pieds, ladite détermination de tous les points de force critiques pour l'utilisateur sur le matelas en fonction des n points de force critiques comprend :
la détermination d'un rapport de jambe à corps pour l'utilisateur en fonction de son information préstockée dans la borne ;
la détermination du point de force de hanche en fonction du rapport de jambe à corps et des positions du point de force de tête et du point de force de pieds ;
la détermination d'une zone prédéfinie des deux côtés de la distance depuis le point de force de tête jusqu'au point de force de hanche comme étant des plages de force des bras ; et
la détermination du point de force de tête, des points de force de bras, du point de force de hanche et du point de force de pieds comme étant tous les points de force critiques pour l'utilisateur sur le matelas.

5. Procédé selon la revendication 3, **caractérisé en ce que** si n = 3 et les n points de force critiques sont le point de force de tête, le point de force de pieds et le point de force de hanche, ladite détermination de tous les points de force critiques pour l'utilisateur sur le matelas en fonction des n points de force critiques comprend :
la détermination d'une zone prédéfinie des deux côtés de la distance depuis le point de force de tête jusqu'au point de force de hanche comme étant les plages de force des bras ; et
la détermination du point de force de tête, des points de force de bras, du point de force de hanche et du point de force de pieds comme étant tous les points de force critiques pour l'utilisateur sur le matelas.

6. Procédé selon la revendication 3, **caractérisé en ce que** si n = 5 et les n points de force critiques sont le point de force de tête, le point de force de pieds, le point de force de hanche et le point de force de bras, ladite détermination de tous les points de force critiques pour l'utilisateur sur le matelas en fonction des n points de force critiques comprend :
la détermination de plages respectives depuis les deux points de force de bras jusqu'au point de force de tête comme étant les points de force de bras ; et
la détermination du point de force de tête, des points de force de bras, du point de force de hanche et du point de force de pieds comme étant tous les points de force critiques pour l'utilisateur sur le matelas.

7. Procédé selon la revendication 1, **caractérisé en ce que** ledit envoi de l'information d'exercice incluant le statut d'exercice à la borne comprend :
la production d'une information d'exercice incluant le statut d'exercice, et l'envoi de l'information d'exercice à la borne, qui détermine les parties d'entraînement de corps de l'utilisateur en fonction du statut d'exercice ; ou
l'acquisition du nombre des mouvements du statut d'exercice, la production de l'information d'exercice incluant le statut d'exercice et le nombre de mouvements, et l'envoi de l'information d'exercice à la borne, qui calcule une énergie totale qu'il en coûte à l'utilisateur sur le matelas en fonction du nombre de mouvements et de la consommation d'énergie d'un unique mouvement.

8. Dispositif pour un enregistrement d'exercices, qui s'applique à un matelas qui présente des capteurs de pression répartis sur celui-ci et est relié à une borne sans fil, le dispositif comprenant :
un module de détermination de points de force (310), configuré pour déterminer des points de force critiques pour un utilisateur sur le matelas, dans lequel lesdits points de force critiques sont les points du corps de l'utilisateur qui touchent le matelas lorsque l'utilisateur réalise des exercices physiques ;
un module de détermination de statut (320), configuré pour déterminer un statut d'exercice de l'utilisateur sur le matelas en fonction d'une différence de valeurs de pression des capteurs de pression en différents points de force critiques ; et
un module d'envoi d'informations (330), configuré pour envoyer l'information d'exercice à une borne, l'information d'exercice incluant le statut d'exercice, ledit dispositif étant **caractérisé en ce que** le module de détermination de statut comprend :
un sous-module de mesure de pression (421), configuré pour mesurer périodiquement les valeurs de pression des capteurs de pression au niveau de chaque point de force critique ; et
un sous-module de détermination de statut (422), configuré pour trouver une règle de variation de pression en chaque point de force critique, et rechercher le statut d'exercice correspondant à la règle de variation de pression dans une relation de correspondance prédéfinie qui est stockée dans le matelas, où une relation de correspondance entre un statut d'exercice individuel et une règle de variation individuelle de pression est stockée dans la relation de correspondance prédéfinie,
dans lequel :
a/ si la variation de pression est déterminée comme suit :
la valeur de pression du point de force de tête passe d'une pression inchangée à zéro,
la valeur de pression du point de force de hanche augmente,
la valeur de pression du point de force critique de pieds demeurent inchangée, et
les valeurs de pression des points de force de bras demeurent à zéro,
le statut d'exercice est déterminé comme étant le fait de s'asseoir depuis une position allongée ;
b/ si la variation de pression est déterminée comme suit :
les valeurs de pression des points de force de bras demeurent inchangées,
les valeurs de pression des points de force de tête demeurent inchangées,
les valeurs de pression du point de force de hanche passent d'inchangées à zéro, et
la valeur de pression du point de force de pieds demeure à zéro,
le statut d'exercice est déterminé comme étant le fait de soulever une jambe depuis une position allongée ;
c/ si la variation de pression est déterminée comme suit :
les valeurs de pression du point de force de tête demeurent à zéro,
les valeurs de pression du point de force de hanche demeurent à zéro,
les valeurs de pression des points de force de bras sont augmentées, et
la valeur de pression du point de force de pieds est augmentée,
le statut d'exercice est déterminé comme étant le fait de s'appuyer depuis une position prédisposée pour faire des pompes.

9. Dispositif selon la revendication 8, **caractérisé en ce que** le module de détermination de points de force comprend :
un premier sous-module de détermination (411), configuré pour déterminer n points de force critiques pour l'utilisateur sur le matelas en fonction des valeurs de pression des capteurs de pression lorsque l'utilisateur est allongé sur le matelas, dans lequel n ≥ 2 ; et
un second sous-module de détermination (412), configuré pour déterminer tous les points de force critiques pour l'utilisateur sur le matelas en fonction des n points de force critiques déterminés par le premier sous-module de détermination.

10. Dispositif selon la revendication 9, **caractérisé en ce que** le premier sous-module de détermination est en outre configuré pour :
si n = 2, le fait de trouver, dans la longueur du matelas, deux points de force critiques qui sont de la distance la plus longue de l'un à l'autre et où les valeurs de pression des capteurs ne sont pas zéro ; et la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ;
si n = 3, le fait de trouver, dans la longueur du matelas, deux points de force critiques qui sont de la distance la plus longue de l'un à l'autre et où les valeurs de pression des capteurs ne sont pas zéro ; la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ; et la détermination d'un point de force qui est de la valeur de pression la plus grande du capteur de pression comme étant le point de force de hanche ;
si n = 5, le fait de trouver, dans la longueur du matelas, deux points de force critiques qui sont de la distance la plus longue de l'un à l'autre et où les valeurs de pression des capteurs ne sont pas zéro ; la détermination de leur point de force avec la valeur de pression la plus grande des capteurs de pression comme étant le point de force de tête, et la détermination de leur point de force avec la valeur de pression la plus petite des capteurs de pression comme étant le point de force de pieds ; la détermination d'un point de force qui est de la valeur de pression la plus grande du capteur de pression comme étant le point de force de hanche ; le fait de trouver, dans la largeur du matelas, deux points de force critiques qui sont de la distance la plus longue de l'un à l'autre et où les valeurs de pression des capteurs ne sont pas zéro, et la détermination des deux points de force critiques comme étant deux points de force de mains.

11. Dispositif selon la revendication 10, **caractérisé en ce que** si n = 2 et les n points de force critiques sont le point de force de tête et le point de force de pieds, le second module de détermination est en outre configuré pour :
déterminer un rapport de jambe à corps pour l'utilisateur en fonction de son information préstockée dans la borne ;
déterminer le point de force de hanche en fonction du rapport de jambe à corps et des positions du point de force de tête et du point de force de pieds ;
déterminer une plage prédéfinie des deux côtés de la distance depuis le point de force de tête jusqu'au point de force de hanche comme étant des plages de force des bras ; et
déterminer le point de force de tête, les points de force de bras, le point de force de hanche et le point de force de pieds comme étant tous les points de force critiques pour l'utilisateur sur le matelas.

12. Dispositif selon la revendication 8, **caractérisé en ce que** le module d'envoi d'informations comprend :
un premier sous-module d'envoi (431), configuré pour produire une information d'exercice incluant le statut d'exercice, et envoyer l'information d'exercice à la borne, qui détermine les parties d'entraînement de corps de l'utilisateur en fonction du statut d'exercice ; ou
un second sous-module d'envoi (432), configuré pour acquérir le nombre de mouvements du statut d'exercice, produire l'information d'exercice incluant le statut d'exercice et le nombre de mouvements, et envoyer l'information d'exercice à la borne, qui calcule une énergie totale qu'il en coûte à l'utilisateur sur le matelas en fonction du nombre de mouvements et de la consommation d'énergie d'un unique mouvement.
